# EUROPÄISCHE PATENTSCHRIFT

(11) **EP 4 281 178 B1**
(45) Veröffentlichungstag und Bekanntmachung des Hinweises auf die Patenterteilung: **25.02.2026**
(21) Anmeldenummer: 22702417.1
(22) Anmeldetag: 18.01.2022
(51) Int. Cl.: A61N 5/06, A61F 5/41

(54) **PENISRING**
COCK RING
ANNEAU PÉNIEN

(30) Priorität: 22.01.2021 DE 202021100303 U; 20.08.2021 DE 202021104463 U
(43) Veröffentlichungstag der Anmeldung: 29.11.2023
(73) Patentinhaber: MEDLight GmbH, 32051 Herford (DE)
(72) Erfinder: WÄCHTER, Herta, 32105 Bad Salzuflen (DE); STOLZE, Jürgen, 41541 Dormagen (DE)
(74) Vertreter: Schleitzer, Dirk-Karsten
(86) Internationale Anmeldenummer: PCT/EP2022/050963
(87) Internationale Veröffentlichungsnummer: WO 2022/157136

(56) Entgegenhaltungen:
- WO-A1-2010/078929
- KR-A- 20060 127 450
- KR-A- 20120 017 108
- KR-A- 20150 080 411
- KR-B1- 101 786 423
- US-A1- 2005 197 682
- US-A1- 2007 179 337
- US-A1- 2009 112 055
- US-A1- 2014 303 693
- US-A1- 2017 259 079
- KIM TAEUK ET AL: "A Synthetic Erectile Optogenetic Stimulator Enabling Blue-Light-Inducible Penile Erection", ANGEWANDTE CHEMIE INTERNATIONAL EDITION, vol. 54, no. 20, 18 March 2015 (2015-03-18), pages 5933 - 5938, XP055913504, ISSN: 1433-7851, DOI: 10.1002/anie.201412204

## Beschreibung

Die Erfindung betrifft einen Penisring, insbesondere zur sexuellen Stimulation und/oder zur Förderung der Erektion des Mannes, besonders bevorzugt zur Erhöhung der Spermienaktivität, mit einem ringförmigen Behandlungskörper zum Umgreifen des Penis.

Bei sogenannten "Penisringen", die auch unter der Bezeichnung "Intim Ring" bekannt sind, handelt es sich um Erotikspielzeuge, die nach üblicher Bauart aus einem ringartigen Grundkörper bestehen und damit über das Glied überstreifbar sind, insbesondere derart, dass der Blutfluss im Schwellkörper beeinflusst bzw. gestaut wird und dadurch die Erektion intensiviert und/oder verlängert wird. Insbesondere werden Penisringe bzw. Intim Ringe verwendet, um bei sexuellen Handlungen eine zusätzliche stimulierende Wirkung zu erzeugen oder um das männliche Glied durch erektile Stimulation auf den sexuellen Akt vorzubereiten.

In diesem Zusammenhang geht aus der DE 44 09 721 A1 ein Penisring der eingangs genannten Art hervor, der an wechselnde (Umfangs-)Größen anpassbar ist. So wird vorgeschlagen, einen ersten Ring mit einem zweiten, engeren Ring über ein Lederband zu verbinden und hierdurch eine individuelle Größenanpassung vorzunehmen. Die Stimulation des Penis erfolgt dann ausschließlich durch das Umgreifen bzw. rein mechanisch.

Die US 2007/0179337 A1 offenbart eine Beleuchtungsvorrichtung und Dehnvorrichtung für das männliche Genital. An einer Montiervorrichtung ist dabei eine Vielzahl von Beleuchtungsquellen angebunden.

Die US 2014/0303693 A1 offenbart eine Vorrichtung zur Fototherapie zur Beleuchtung von Licht ausgehend von einer Vielzahl von Lichtquellen. Die Lichtquellen sind innenseitig an einem Schlauch, an einem Behälter oder einem helixförmigen Band angeordnet.

Die US 2009/0112055 A1 offenbart einen Schlauch, an dessen Innenseite eine Vielzahl von Leuchtdioden angeordnet ist.

Aufgabe der vorliegenden Erfindung ist es, einen Penisring der zuvor beschriebenen Art bereitzustellen, mit dem es möglich ist, die Durchblutung eines Gewebes zu fördern und damit, vorzugsweise, eine antiseptische Wirkung zu erreichen. Insbesondere ist es Aufgabe der vorliegenden Erfindung, einen Penisring bereitzustellen, der über eine rein mechanisch initiierte Stimulation aufgrund des Umgreifens eine weitere Maßnahme zur Intensivierung der Stimulation und/oder zur Entfaltung einer zusätzlichen therapeutischen Wirkung vorschlägt, wobei der Penisring als kompaktes Gerät für den Verbraucher und für die Behandlung bzw. Therapie in häuslicher Umgebung vorgesehen ist. Insbesondere soll die Anwendung eines Penisrings realisiert werden, die mit einer Erhöhung der Spermienaktivität, also einer die Potenz des Mannes positiv beeinflussenden Wirkung, einhergeht. Insofern soll neben einer reinen Stimulationswirkung auch eine therapeutische Wirkung, insbesondere als Haupt- und/oder Begleittherapie im Rahmen der Potenzsteigerung, zur Verfügung gestellt werden. Bei der bestimmungsgemäßen Verwendung, also wenn der Behandlungskörper den Penis umgreift, wird dieser temporär bis zum Erreichen einer erwünschten Erektionswirkung und/oder über eine festgelegte Therapiedauer zumindest teilweise, vorzugsweise vollständig, am Penis belassen, wobei sich der Penisring durch einen angenehmen Tragezustand auszeichnen und auch geeignet sein soll, beim Träger angenehme Reaktionen bis hin zur sexuellen Stimulation bzw. dauerhaften und intensiven Erektion auszulösen.

Die vorgenannte Aufgabe wird durch einen Penisring mit den Merkmalen von Anspruch 1 gelöst. Vorteilhafte Ausgestaltungen der Erfindung sind Gegenstand der abhängigen Ansprüche.

Erfindungsgemäß ist vorgesehen, dass der Penisring eine in dem Behandlungskörper integrierte Bestrahlungseinrichtung mit wenigstens einem Leuchtmittel, insbesondere LED-Leuchtmittel, mit einer integrierten Stromquelle, insbesondere einem integrierten Akkumulator, zur Stromversorgung der Bestrahlungseinrichtung und eine vorzugsweise in den Behandlungskörper integrierte Steuerschaltung zur Steuerung der Bestrahlungseinrichtung aufweist, wobei die Bestrahlungseinrichtung konfiguriert ist zur Emission von blauem und/oder blauviolettem Licht im sichtbaren Wellenlängenbereich mit einer Wellenlänge zwischen 410 nm und 450 nm.

Der Erfindung liegt der Grundgedanke zugrunde, einen Penisring zur sexuellen Stimulation und/oder zur Förderung der Erektion des Mannes einzusetzen, wobei die Lichtemission eines oder mehrerer Lichtmittel des Penisrings erfindungsgemäß in einem Bereich liegen soll, der geeignet ist, eine besonders ausgeprägte und/oder intensive Stimulation bzw. Förderung der Erektion des Mannes hervorzurufen, insbesondere unter vorzugsweise gleichzeitiger Entfaltung einer therapeutischen Wirkung, insbesondere einer Erhöhung der Spermienbeweglichkeit bzw. einer Potenzsteigerung des Mannes. Der erfindungsgemäße Penisring verstärkt somit die herkömmliche Stimulation infolge des Umgreifens bzw. Abschnürens bei Anwendung eines Penisrings durch eine gezielte Bestrahlung mit blauem und/oder blauviolettem Licht im sichtbaren Wellenlängenbereich mit einer Wellenlänge zwischen 410 nm und 450 nm. Dies wurde im Rahmen praktischer Erprobungen bestätigt, wonach von Probanden durch Anwendung des erfindungsgemäßen Penisring mit der vorgesehenen Bestrahlung im Vergleich zu herkömmlichen Penisringen, also durch rein mechanische Stimulation, eine deutliche erhöhte Stimulationswirkung, insbesondere eine intensiver ausgeprägte und/oder länger anhaltende Erektion, berichtet wurde. Es ist in diesem Zusammenhang davon auszugehen, dass die Verwendung des erfindungsgemäßen Penisrings unter Anwendung von Lichtemission im gewählten Bereich zu einer weiteren Anregung des Blutflusses bzw. der Blutzirkulation führt, einhergehend mit einer besonders intensiven Stimulation und/oder Erektionsausprägung.

In der Ausgestaltung als Penisring ist vorzugsweise ein lockerer Sitz am Penis vorgesehen, so dass es nicht zu einem Blutstau kommen kann. Damit unterscheidet sich dann der erfindungsgemäße Penisring von aus dem Stand der Technik bekannten Penisringen, die einen Blutstau bewirken sollen. Allerdings ist nicht grundsätzlich ausgeschlossen, dass es auch beim Tragen des erfindungsgemäßen Penisrings zu einem Blutstau kommt.

Überdies wurde in überraschender Weise festgestellt, dass die Bestrahlung im erfindungsgemäß vorgesehenen Sinn auch einen zumindest kurzfristig feststellbaren Einfluss auf die Spermienaktivität bzw. Qualität hat. So wies das Ejakulat, erhalten aus einem mit dem erfindungsgemäßen Penisring behandelten bzw. bestrahlten Gliedes, gegenüber nicht mit dem erfindungsgemäßen Penisring behandeltem Ejakulat eine erhöhte Spermienaktivität auf. In diesem Zusammenhang ist davon auszugehen, dass die durch die Bestrahlung im vorgegebenen Wellenlängenbereich eingeleitete Strahlungsenergie zumindest zu einem gewissen Teil durch die Spermien bzw. Samenzellen aufgenommen bzw. absorbiert wird, was zumindest kurzfristig mit einer Steigerung der Spermienaktivität einhergeht. Insofern eignet sich der erfindungsgemäße Penisring, insbesondere zusätzlich zur zuvor beschrieben weiteren Erektionsintensivierung, auch zur Durchführung therapeutischer Zielsetzungen, die das Ziel haben, eine Potenzsteigerung des Mannes zu bewirken.

Was die Bestrahlung weiterhin anbelangt, so erfolgt dieser vorzugsweise mit sehr geringen Bestrahlungsstärken bzw. Fluenzraten, wobei die Bestrahlungsstärke in der Einheit [W/cm²] angegeben wird und die durch Strahlung übertragene Energie in [J] bezogen auf die Empfängeroberfläche des durch den Behandlungskörper umschlossenen Gliedes und die Zeit beschreibt. Die Empfängeroberfläche bzw. Behandlungsoberfläche entspricht dabei insbesondere aufgrund der direkten Anlage des Penisrings einer Kontaktfläche, mit welcher das Innere des Penisrings gegen das umschlossene Glied unmittelbar aufliegt. Erfindungsgemäß erfolgt eine Beleuchtung mit niedrigen Bestrahlungsstärken, so dass die Beleuchtung über einen relativ langen Zeitraum erfolgt, um eine gewünschte Lichtdosis zu erreichen, die zur Erzielung einer Stimulation und/oder therapeutischen Wirkung erforderlich ist. Durch die Beleuchtung mit niedrigen Bestrahlungsstärken wird verhindert, dass bei der Anwendung des Penisrings Schmerzen, beispielsweise in Form von Hautirritationen, auftreten, woraus in Verbindung mit einer individuellen Einstellung der Öffnungsweite ein angenehmer Tragezustand resultieren kann. Dadurch wird es hochwahrscheinlich, dass regelmäßige, freiwillige Behandlungen stattfinden.

Mit der Erfindung wird insbesondere ein Penisring zur Anwendung als Vergnügungsspielzeug für eine kombinierte Stimulations- und Potenzsteigerungs-Therapie vorgeschlagen, wobei eine kontrollierte Strahlungsenergie auf die durch den Behandlungskörper umschlossene (Vor-)Haut abgegeben wird, was, insbesondere in Kombination mit der durch den Penisring verbundenen mechanischen Einwirkung infolge des Umgreifens, zu einer besonders intensiven Stimulation und/oder Förderung der Erektion führt und, insbesondere zusätzlich oder alternativ, einen zumindest kurzfristig positiven therapeutischen Einfluss auf die Spermienaktivität hat.

Besonders positive Ergebnisse in Bezug auf die Stimulations- und Potenzsteigerungs-Therapie im obigen Sinne werden bei der Emission von insbesondere blauem und/oder blauviolettem Licht im sichtbaren Wellenlängenbereich mit einer Wellenlänge zwischen 410 nm und 450 nm erzielt.

Das Bestrahlen des umschlossenen Penis(-abschnitts) mit Licht im erfindungsgemäß vorgesehenen Wellenlängenbereich führt dabei, ohne sich auf eine bestimmte Theorie festlegen zu wollen, zu einem teilweisen Eindringen von Lichtenergie in das Penisinnere, nämlich in den Schwellkörper und/oder in die Harnröhre, so dass eine Behandlung im angestrebten Sinne möglich ist, ohne empfindliche Strukturen zu beschädigen. Laut Studien kann die Lichtstrahlung zwischen 1 bis 2 mm in den Penis eindringen, wobei ein tieferes Eindringen aufgrund der dünnen Hautschicht im Penisbereich durchaus möglich ist.

Mit der Erfindung wird somit der Anwendungsbereich von Penisringen über die bekannte Verwendung zur Stimulation, die auf das rein mechanische Umschließen zurückzuführen ist, intensiviert, aber auch erweitert auf einen therapeutischen Anwendungsbereich. So lässt sich der erfindungsgemäße Penisring insbesondere zur Potenzsteigerung, insbesondere zur Erhöhung der Spermienaktivität und/oder Spermienqualität, durch gezielte Bestrahlung einsetzen.

Als Lichtemitter bzw. Leuchtmittel für die Bestrahlung ist vorzugsweise wenigstens eine lichtemittierende Diode (LED) vorgesehen, was die Bestrahlung mit einer definierten und erfindungsgemäßen Wellenlänge ermöglicht und die Verwendung von beispielsweise optischen Filtern, um bestimmte ungewünschte Wellenlängen herauszufiltern und eine Bestrahlung im angestrebten Wellenlängenbereich sicherzustellen, entbehrlich macht. LEDs sind dabei insbesondere punktförmige Lichtquellen und haben im Vergleich zu anderen Leuchtmitteln keine ausgeprägte Rundum-Abstrahlung, strahlen also mit einem vergleichsweise geringen Abstrahlwinkel, beispielsweise von weniger als 180°, ab. Die punktförmige Abstrahlung ist im Sinne der vorliegenden Erfindung dabei besonders bevorzugt, um die Einleitung des Lichtes in vorgegebenen Wellenlängenbereich besonders intensiv und fokussiert vorzugnehmen. Um trotz des begrenzten Abstrahlwinkels punktförmiger Leuchtmittel, wie LEDs, eine vollständige umfangseitige Bestrahlung zu realisieren kommen erfindungsgemäß besonders bevorzugt mehrere Lichtmittel, insbesondere LEDs, zum Einsatz, um eine Bestrahlung des Penis vorzugsweise um den gesamten Umfang herum zu ermöglichen.

Die Bestrahlungsstärke ist erfindungsgemäß bezogen auf einen Zustand, in dem der Bestrahlungskörper mit seiner inneren Ringfläche, insbesondere vollumfänglich und/oder vollflächig, gegen das umschlossene Glied anliegt.

Im Übrigen kann der erfindungsgemäße Penisring eine Datenkommunikationseinrichtung aufweisen, die zum Senden und/oder Empfangen von Funksignalen ausgebildet ist und einen Datentransfer mit einer entfernten Bedieneinrichtung zur Steuerung der Bestrahlungseinrichtung ermöglicht. Die Datenkommunikationseinrichtung kann ein Antennenelement zum Senden und/oder Empfangen von Funksignalen umfassen. Das Antennenelement kann für das Senden und/oder Empfangen von Funksignalen nach Bluetooth, NFC (Near Field Communication), WLAN, GSM, UMTS, LTE oder einem anderen mobilen Datenübertragungsstandard geeignet sein.

Es versteht sich, dass die angegebenen Wellenlängenangaben bezogen auf eine zulässige Peakabweichung von ± 3 nm zu verstehen sind. In Zusammenhang mit der Erfindung durchgeführte Versuche an Probanden haben gezeigt, dass sich in diesem Wellenlängenspektrum eine sehr erfolgversprechende Stimulation des Schwellkörpers und/oder Aktivierung der Samenzellen erreichen lässt.

Die Bestrahlungseinrichtung ist darüber hinaus vorzugsweise zur Beleuchtung mit einer Bestrahlungsstärke zwischen 0,1 mW/cm² und 2,0 mW/cm², vorzugsweise zwischen 0,25 und 1,0 mW/cm², konfiguriert, bezogen auf die Kontaktfläche bzw. Behandlungsfläche, mit welcher der Penisring im Anwendungszustand das männliche Glied umschließt. Durch diese vorzugsweise vorgesehene Bestrahlungsstärke wird eine optimale Stimulation bzw. Steigerung der Erektion und/oder der Spermienaktivität erzielt, ohne unerwünschte Hautreizungen und/oder Schädigungen im Bereich der Anwendungsfläche oder im Penisinneren zu riskieren, so dass mit dem erfindungsgemäßen Penisring eine schmerzfreie und sichere Anwendung gewährleistet ist.

Um weiterführend in diesem Zusammenhang (Haut-)Schädigungen sicher ausschließen zu können, ist die Steuerschaltung bei einer bevorzugten Ausführungsform der Erfindung zum automatischen Abschalten der Bestrahlung bei Erreichen einer Bestrahlungsenergie von weniger als 5,0 J, weiter vorzugsweise von weniger als 2,0 J, besonders bevorzugt von 0,5 bis 1,5 J, und/oder nach einer Bestrahlungsdauer von höchstens 60 min, vorzugsweise von beispielsweise höchstens 30 min, weiter vorzugsweise von höchstens 20 Minuten, ausgebildet. Die Bestrahlungsdauer kann hierbei von einer Person frei oder in diskreten Schritten innerhalb vorgegebener Werte einstellbar sein oder es kann wenigstens eine feste Beleuchtungsdauer unveränderbar vorgegeben und in der Steuerschaltung hinterlegt sein.

Durch entsprechende Auslegung des Leuchtmittels und/oder der Anzahl der Leuchtmittel lässt sich die Oberflächentemperatur und die Bestrahlungsstärke des Bestrahlungskörpers begrenzen, die beim Betrieb der Bestrahlungseinrichtung maximal erreicht werden soll. Es ist zweckmäßig, wenn die Oberflächentemperatur des Behandlungskörpers, insbesondere auf einer dem Penis zugewandten inneren Ringfläche, bei unterbrechungsloser Bestrahlung des umgriffenen Gliedabschnitts mit einer maximalen Bestrahlungsstärke über einen Zeitraum von weniger als 60 min, vorzugsweise von weniger als 30 Minuten, weiter vorzugsweise von weniger als 20 Minuten, einen Maximalwert von 45 °C, vorzugsweise von 42 °C, weiter vorzugsweise von 40 °C, nicht überschreitet. Dies erfordert eine Abstimmung der Bestrahlungsstärke der Bestrahlungseinrichtung, der Wärmespeicherkapazität des Behandlungskörpers und der integrierten Bauteile, insbesondere der Steuerschaltung, sowie der vorgesehenen Zeitdauer der Bestrahlung.

Die Lichtemission kann kontinuierlich erfolgen oder die Bestrahlungseinrichtung wird gepulst betrieben. Beim gepulsten Betrieb wird das Licht in zeitlich begrenzten Portionen (Pulsen) emittiert, wobei die Pulslänge so gewählt sein kann, dass der Behandlungskörper an seiner Oberfläche einen Maximalwert von 45 °C, vorzugsweise von 42 °C, weiter vorzugsweise von 40 °C, nicht überschreitet.

Im Übrigen kann die Steuerschaltung konfiguriert sein zum automatischen Abschalten der Bestrahlung nach Erreichen einer Bestrahlungsdauer von weniger als 60 min, vorzugsweise von weniger als 30 min, weiter vorzugsweise von weniger als 20 min, und/oder bei Erreichen einer emittierten Bestrahlungsenergie von weniger als 5,0 J/cm², weiter vorzugsweise von weniger als 2,0 J/cm², besonders bevorzugt von 0,5 J/cm² bis 1,5 J/cm².

Vorzugsweise ist eine Anordnung des Leuchtmittels und/oder ein Abstrahlwinkel des Leuchtmittels vorgesehen, die bzw. der eine radial nach innen gerichtete und/oder in das Zentrum des Behandlungskörpers gerichtete Bestrahlung ermöglicht. Generell ist im Rahmen der Bestrahlung vorzugsweise angestrebt, dass die abgegebene Strahlungsenergie im Wesentlichen bzw. mit ihrem Hauptanteil radial nach innen, bezogen auf den ringförmigen Behandlungskörper, abgestrahlt wird, um eine besonders effiziente Bestrahlung des umschlossenen Penisbereichs mit dem Licht im vorgesehenen Spektrum zu ermöglichen.

Der Begriff "Abstrahlwinkel" beschreibt in diesem Zusammenhang die winkelabhängige Lichtstärke. Im Falle gerichteter, vorzugsweise punktförmiger, Lichtquellen ist der Abstrahlwinkel ein Winkel, der von den seitlichen Punkten, mit der das Licht vom Leuchtmittel abstrahlt, noch mit halber Maximal-Lichtstärke eingeschlossen wird. Der Abstrahlwinkel gibt also an, in welchem Winkel das Licht nach vorne abgestrahlt wird. Wie bereits erwähnt werden im Rahmen der vorliegenden Erfindung vorzugsweise LEDs als Lichtmittel eingesetzt, die sich insbesondere durch eine punktförmige Lichtquelle und einen vergleichsweise geringen Abstrahlwinkel auszeichnen. Besonders bevorzugt werden dabei LEDs mit einem Abstrahlwinkel zwischen 10° und 120° eingesetzt. Um auch beim besonders bevorzugten Einsatz von mehreren Lichtmitteln, insbesondere LEDs, eine weitreichende Bestrahlung des Penisumfangs zu ermöglichen, werden vorzugsweise Lichtmittel bzw. LEDs mit demselben Abstrahlwinkel eingesetzt, wobei die Vorschrift 360°/n gilt, wobei n die Anzahl der eingesetzten Lichtmittel in Umfangsrichtung entspricht. Dadurch lässt sich eine besonders effiziente und in Umfangsrichtung vollständige Bestrahlung des Penisabschnitts realisieren.

Die volle Lichtstärke befindet sich dabei im Zentrum des von dem Leuchtmittel bzw. der LED erzeugten Lichtkreises, wobei bei einer bevorzugten Ausführungsform der Erfindung die maximale Lichtstärke in einer Richtung senkrecht zur inneren Umfangsfläche des Behandlungskörpers und in Richtung des Zentrums des Behandlungskörpers abgestrahlt wird.

Zur Stromversorgung kann wenigstens ein vorzugsweise austauschbarer und/oder wieder aufladbarer Speicher für elektrische Energie vorgesehen sein, insbesondere ein Speicher für elektrische Energie auf elektrochemischer Basis, weiter insbesondere eine herkömmliche Batterie, und/oder es kann eine Einrichtung zum kabellosen induktiven Wiederaufladen des Speichers vorgesehen sein.

Vorzugsweise weist die Bestrahlungseinrichtung mehrere Leuchtmittel, insbesondere LEDs, auf.

Sind mehrere Leuchtmittel vorgesehen, so können diese in Umfangsrichtung, vorzugsweise gleichverteilt, entlang einer inneren Ringfläche des Behandlungskörpers angeordnet sein. Der Begriff "innere Ringfläche" beschreibt dabei insbesondere jenen Teil der Oberfläche des Penisrings, der dem Penis zugewandt ist und, insbesondere, im Anwendungszustand unmittelbar mit diesem in Kontakt steht. Die innere Ringfläche setzt sich vorzugsweise aus zwei Teilflächen zusammen, nämlich aus einer vorzugsweise lichtundurchlässigen Kontaktfläche einerseits, über die kein Lichtaustrag erfolgt, und aus wenigstens einer Lichtaustrittsfläche, und über welche die Emission des erfindungsgemäß vorgesehenen Lichtes stattfindet und die somit dem Leuchtmittel zugeordnet oder durch dieses gebildet ist. Vorzugsweise sind mehrere Lichtaustrittsflächen vorgesehen, die durch die Kontaktfläche umschlossen werden.

Besonders bevorzugt ist in diesem Zusammenhang vorgesehen, dass die Leuchtmittel in Umfangsrichtung, vorzugsweise gleichverteilt, über die gesamte innere Ringfläche des Behandlungskörpers angeordnet sind. Dementsprechend erfolgt vorzugsweise eine in sich geschlossene bzw. unterbrechungsfreie Bestrahlung über den gesamten Umfang des Gliedes, um die Intensität der angestrebten Wirkungen dementsprechend zu maximieren.

Besonders bevorzugt sind die Leuchtmittel in diesem Zusammenhang dabei bezüglich ihres Punktes der vollen bzw. maximalen Lichtstärke in Umfangsrichtung, vorzugsweise gleichverteilt, über die gesamte innere Ringfläche des Behandlungskörpers angeordnet. Gleichverteilt bedeutet in diesem Zusammenhang insbesondere, dass zwischen unmittelbar aufeinander folgenden Leuchtmitteln ein Versatzwinkel vorliegt, welcher die Vorschrift 360°/n, wobei n die Anzahl der eingesetzten Leuchtmitteln sind, erfüllt.

Vorzugsweise weist der Behandlungskörper auf der inneren Ringfläche wenigstens eine Lichtöffnung zur insbesondere passgenauen Aufnahme des wenigstens einen Leuchtmittels auf. Nicht ausgeschlossen ist jedoch, dass der Behandlungskörper oder ein Gehäuse bzw. Gehäuseteil des Behandlungskörpers für eine Lichtabgabe nach innen vollständig oder auch lediglich bereichsweise aus einen lichtdurchlässigen Material besteht. In diesem Fall kann der Einsatz einer Lichtöffnung auch entfallen, da die innere Ringfläche durch das Gehäuse oder einen Gehäuseabschnitts des Behandlungskörpers gebildet ist und das wenigstens eine in dem Gehäuse aufgenommene Leuchtmittel über einen transparenten Abschnitt dann radial nach innen abstrahlt.

Was die Dimensionierung der Lichtöffnung anbelangt, so erstreckt sich diese vorzugsweise über einen Bereich von 25 bis 75 %, vorzugsweise über einen Bereich von 40 bis 60 %, bezogen auf die axiale Höhe des Behandlungskörpers. Es ist jedoch auch möglich, dass sich die Lichtöffnung über die gesamte Höhe, also über 100 %, der Höhe, also der axialen Erstreckung, der inneren Ringfläche des Behandlungskörpers erstreckt oder die gesamte innere Ringfläche durch vorzugsweise lediglich eine einzige Lichtöffnung gebildet ist.

Der Penisring weist vorzugsweise eine axiale Höhe im Bereich von 1 bis 10 cm, vorzugsweise 2 bis 8 cm, besonders bevorzugt im Bereich von 3 bis 6 cm, auf.

Besonders bevorzugt ist die wenigstens eine Lichtöffnung auf mittlerer Höhe des Bestrahlungskörpers auf der inneren Ringfläche angeordnet. Dies schließt jedoch eine davon abweichende Anordnung, nämlich in einem oberen oder unteren Randbereich des Bestrahlungskörpers, insbesondere der inneren Ringfläche, nicht aus.

Die Lichtöffnung und/oder eine Lichtaustrittsfläche des Leuchtmittels kann bzw. können sich vorzugsweise über einen Bereich von 25 bis 90 %, vorzugsweise 40 bis 60 %, über den Umfang des Behandlungskörpers, vorzugsweise der inneren Ringfläche, erstrecken. In diesem Zusammenhang ist der Begriff "Umfang" vorzugsweise in Bezug auf die mittlere Axialerstreckung des Behandlungskörpers, also der mittleren axialen Höhe des Behandlungskörpers, insbesondere der inneren Ringfläche, zu verstehen. Dementsprechend werden entlang des Umfangs Bereiche bzw. Abschnitte durch die Lichtöffnung bzw. die Lichtaustrittsfläche gemäß den obigen Prozentangaben eingenommen. Die übrigen Flächenanteile entlang des Umfangs sind dementsprechend geschlossen und/oder nicht zum Lichtaustrag vorgesehen und, vorzugsweise, zumindest im Wesentlichen lichtundurchlässig, also als reine Kontakt-fläche mit der Behandlungsfläche ausgebildet.

Ebenso ist es auch möglich, dass sich eine einzige Lichtöffnung und/oder Lichtaustrittsfläche sich im Wesentlichen über den gesamten Umfang des Behandlungskörpers erstreckt, insbesondere streifen- oder bandförmig. In diesem Fall kann die innere Ringfläche selbst durch die Lichtöffnung und/oder die Lichtaustrittsfläche gebildet sein. Es ist anzumerken, dass bei Einsatz lediglich einer Lichtöffnung und/oder Lichtaustrittsfläche es nicht ausgeschlossen ist, dass mehrere Leuchtmittel eingesetzt werden. So ist es in diesem Zusammenhang insbesondere möglich, dass mehrere Leuchtmittel in einer einzigen Lichtöffnung in Umfangsrichtung, vorzugsweise gleichverteilt, angeordnet sind. Besonders bevorzugt können diese Leuchtmittel dann auch von einem einzigen transparenten Verschlusselement verschlossen sein, über welches dann die Emission des Lichtes erfolgt.

Die Fläche der Lichtöffnung und/oder die Lichtaustrittsfläche kann vorzugsweise zwischen 20 und 80 %, vorzugsweise zwischen 40 und 60 %, der inneren Ringfläche des Behandlungskörpers einnehmen. Dies gewährleistet einerseits eine intensive Bestrahlung des umgriffenen Penisabschnitts, wirkt aber auch der Ausbildung eines unzulässig hohen Energieeintrags und/oder der Ausbildung von Hautirritationen oder sonstigen Schädigungen entgegen.

Vorzugsweise weist der Behandlungskörper, insbesondere auf der inneren Ringfläche, mehrere Lichtöffnungen auf, wobei, vorzugsweise, jedem Leuchtmittel eine separate Lichtöffnung zugeordnet ist. Die Lichtöffnungen können in Umfangsrichtung voneinander beabstandet bzw. voneinander getrennt sein durch Flächenbereiche des Behandlungskörpers, insbesondere durch Kontaktflächen der inneren Ringfläche. Besonders bevorzugt wird die innere Ringfläche dabei durch lichtdurchlässige Flächen, über welche die Lichtemission erfolgt, einerseits sowie lichtundurchlässige oder lichtschwache Kontaktflächen, über welche dann in Wesentlichen keine oder eine abgeschwächte Lichtemission erfolgt, andererseits gebildet.

Was die Anordnung der Lichtöffnungen anbelangt, so sind diese vorzugsweise über den gesamten Umfang des Behandlungskörpers, vorzugsweise über den gesamten Umfang der inneren Ringfläche, verteilt, vorzugsweise gleichverteilt.

Dementsprechend können mehrere Lichtmittel der Anzahl n vorgesehen sein, die in Umfangsrichtung gleichbleibend um einen Versatzwinkel von 360°/n zueinander versetzt sind. Sind beispielsweise 12 Leuchtmittel vorgesehen, ergibt sich daraus ein Versatzwinkel von 30°. Dabei bezieht sich der Versatzwinkel vorzugsweise auf das Zentrum der Lichtöffnungen. Alternativ oder zusätzlich können sich diese jedoch auch auf den Punkt der maximalen Bestrahlung des Leuchtmittels beziehen, der im Optimalfall im Wesentlichen dem Zentrum der Lichtöffnung entspricht, jedoch auch von diesem
abweichen kann.

Die Lichtöffnungen können sich in Summe über einen Anteil von 30 bis 80 %, vorzugsweise von 40 bis 70 %, insbesondere von 50 bis 60 %, entlang des Umfangs des Behandlungskörpers, vorzugsweise der inneren Ringfläche, erstrecken. In Bezug auf die Definition "Umfang", wie sie im Rahmen der vorliegenden Erfindung vorzugsweise verwendet wird, darf auf die vorherige Ausführung in Bezug auf die Erstreckung des Lichtmittels verwiesen werden, wonach es sich vorzugsweise um den Umfang bezüglich der mittleren axialen Höhe des Behandlungskörpers, insbesondere der inneren Ringfläche, handelt.

Insbesondere korrespondieren die Erstreckungen der Lichtöffnungen zu den Erstreckungen der Leuchtmittel, die insbesondere passgenau in zugeordnete Lichtöffnungen aufgenommen sind.

Vorzugsweise nimmt die Fläche aller Lichtöffnungen zwischen 20 und 80 %, vorzugsweise zwischen 40 und 60 %, einer Ringfläche des Behandlungskörpers, vorzugsweise der inneren Ringfläche des Behandlungskörpers, ein. Auf diese Weise lassen sich Vorteile in Bezug auf eine effiziente Bestrahlung zur Erzielung der gewünschten Wirkungen erzielen.

Was die Ausbildung der Lichtöffnung und/oder der Lichtaustrittsfläche des Leuchtmittels anbelangt, so kann diese langgestreckt und/oder bandförmig ausgebildet sein.

Auch sind mehrere axial übereinanderliegende Reihen von Lichtöffnungen und/oder Leuchtmitteln möglich. Beispielsweise kann auch eine spiralförmige Ausbildung, also ein gewendelter Verlauf einer vorzugsweise einzigen Lichtöffnung oder mehrere aneinander gereihter Lichtöffnungen mit darin aufgenommenen Leuchtmitteln, möglich sein.

Alternativ oder zusätzlich kann die Lichtöffnung jedoch auch rund, insbesondere kreisförmig, oval oder punktförmig ausgebildet sein. Es versteht sich, dass entlang des Umfangs, beispielsweise alternierend, unterschiedlich ausgebildete Lichtöffnungen vorgesehen sein können, beispielweise eine abwechselnde Anordnung von runden, insbesondere kreisförmigen, und länglichen bzw. bandförmigen Leuchtmitteln, Lichtöffnungen und/oder Lichtaustrittsflächen. Bevorzugt ist jedoch eine einheitliche Ausbildung von Leuchtmitteln und zugeordneten Lichtöffnungen und Lichtaustrittsflächen über den Umfang des Behandlungskörpers vorgesehen.

Zum Verschließen der Lichtöffnung kann ein insbesondere scheibenartiges Verschlussteil aus einem lichtdurchlässigen Material vorgesehen sein. Vorzugsweise ist das Verschlusselement über ein Dichtmittel und/oder Klebmittel am Behandlungskörper, vorzugsweise an der inneren Ringfläche, angebunden und/oder gehalten sein. Das Verschlussteil kann eine ebene oder gekrümmte Oberfläche aufweisen und/oder mit angrenzenden Oberflächen des Behandlungskörpers bzw. der inneren Ringfläche ausgefluchtet sein.

Besonders bevorzugt ist das Verschlusselement gekrümmt, insbesondere derart, dass es einen passgenauen, kreisbogenförmigen Abschnitt des Umfangs der inneren Ringfläche bildet und sich somit unterbrechungsfrei als integraler Teil des Umfangs der inneren Ringfläche einbindet. Dadurch ergibt sich im Anwendungszustand eine erhebungsfreie bzw. unterbrechungsfreie Anschmiegung der inneren Ringfläche, ohne dass für den Anwender spürbare Übergänge zwischen Lichtöffnungen bzw. Lichtaustrittsflächen und daran angrenzende Kontaktflächen vorliegen.

Um eine hohe Wertigkeit der Penisring bzw. eine ästhetisch ansprechende äußere Erscheinungsform der Penisring sicherzustellen, kann das Verschlussteil eine satinierte Oberfläche aufweisen. Durch die satinierte Oberfläche kommt es auch zur Lichtstreuung bei der Bestrahlung, was von Vorteil sein kann.

In einem Gehäuse oder Gehäuseteil des Behandlungskörpers kann ein Leiterplattenaufbau als Teil der Bestrahlungseinrichtung und wenigstens ein Leuchtmittel vorgesehen sein. Vorzugsweise ist ein Gehäuse des Behandlungskörpers zur Aufnahme eines Speichers bzw. Akkumulators für elektrische Energie, insbesondere wenigstens einer Knopfzelle oder Batterie, ausgebildet und weist einen entsprechenden Aufnahmeraum auf.

Der Behandlungskörper kann wenigstens ein Gehäuseteil aus einem vorzugsweise elastischen Kunststoff, insbesondere aus Acrylnitril-Butadien-Styrol-Copolymer (ABS) oder Acrylnitril-Styrol-Acrylat-Copolymer (ASA), aufweisen. Beispielsweise kann der Behandlungskörper aus zwei vorzugsweise identischen ringförmigen Halbschalen gebildet sein, in denen die technischen Komponenten des Penisrings aufgenommen, insbesondere eingekapselt, sind. Ein Verbinden von mehreren Teilen des Penisrings, die die Ringstruktur bilden, ist durch eine Schnapp-, Rast- oder Schraubverbindung beispielsweise möglich.

Das Verschlussteil zum Schließen einer Lichtöffnung kann aus einem transparenten Material, insbesondere aus Kunststoff oder Glas, bestehen, weiter insbesondere aus Polymethylmethacrylat (PMMA) oder ABS und/oder ein solches Material aufweisen. Die Oberfläche des Verschlussteils kann aufgeraut sein. Im Übrigen kann das Verschlussteil als Sammellinse oder Zerstreuungslinse ausgebildet sein und damit insbesondere eine Lupenwirkung erfüllen, was es zulässt, die Leistung der Lichtabgabequelle zu verringern.

Vorzugsweise ist die Lichtöffnung bzw. sind die Lichtöffnungen von einer in sich geschlossenen, integralen Oberfläche bzw. Ummantelung des Behandlungskörpers umgrenzt und/oder umschlossen.

Um eine zielführende Anwendung auch bei unterschiedlichen Penisgrößen, insbesondere Penisumfängen, zu ermöglichen, ist der Behandlungskörper vorzugsweise zumindest teilweise elastisch ausgebildet und/oder flexibel in seiner Öffnungsweite, vorzugsweise elastisch flexibel, einstellbar und/oder aufweitbar ausgebildet.

In diesem Zusammenhang kann der Behandlungskörper eine sich vorzugweise über die gesamte axiale Erhöhung des Behandlungskörpers erstreckende Unterbrechung aufweisen, vorzugsweise wobei die Aussparung zumindest im Anwendungs- bzw. Tragezustand durch zwei aufeinander zuweisende, vorzugsweise abgerundete, freie Enden des Behandlungskörpers seitlich begrenzt ist. Die Unterbrechung ermöglicht die Ausbildung freier Enden, die wiederum durch den Anwender ergriffen und damit eine bedarfsweise und vorzugsweise elastische bzw. federnde Aufweitung des Behandlungskörpers zum Überstreifen des Behandlungskörpers auf das männliche Glied ermöglichen.

Vorzugsweise nimmt die Unterbrechung dabei nur einen vergleichsweise geringen Anteil am vorzugsweise mittleren Gesamtumfang des Behandlungskörpers ein, liegt somit vorzugsweise im Bereich von 1 bis 10 %, vorzugsweise 2 bis 8 %, insbesondere 3 bis 5 %.

Alternativ hierzu kann der Behandlungskörper vorzugsweise auch zwei einander überlappende, gekrümmte Befestigungslaschen aufweisen. Diese Befestigungslaschen sind ebenfalls mit freien Enden versehen, wobei die freien Enden und/oder sonstige Abschnitte der Befestigungslaschen ebenfalls ergriffen werden können, um ein Auseinanderziehen dieser Befestigungslaschen und damit eine Erweiterung der Öffnungsweite des Behandlungskörpers und das Anlegen des Penisrings unter flexibler Einstellung der notwendigen bzw. erwünschten Öffnungsweite zu ermöglichen.

Vorzugsweise erstreckt sich der Überlappungsbereich der gekrümmten Befestigungslaschen über einen Bereich von 10 bis 60 %, vorzugsweise 20 bis 50 %, insbesondere 30 bis 40 %, bezogen auf den vorzugsweise mittleren Gesamtumfang des Behandlungskörpers.

Vorzugsweise sind die Befestigungslaschen lösbar, vorzugsweise form- und/oder reibschlüssig, insbesondere magnetisch, miteinander verbunden.

Die Einstellung der Öffnungsweite kann stufenlos oder stufenweise erfolgen. Insbesondere zur stufenweisen Einstellung der Öffnungsweite kann sich dabei eine Hebelspanneinrichtung als vorteilhaft erweisen, wobei über einen Hebel die freien Enden der Aussparung und/oder der Befestigungslaschen in unterschiedliche Raststellungen zur Festlegung unterschiedlicher Öffnungsweiten bringbar sind.

Ebenfalls kann wenigstens eine Befestigungslasche gelenkig, insbesondere um eine parallel zur Rotationsachse des Behandlungskörpers verlaufende Schwenkachse, ausgebildet sein. Insbesondere die äußere Befestigungslasche ist derart ausgebildet, sodass durch Herausschwenken dieser Befestigungslasche die Überlappung mit der inneren Befestigungslasche auflösbar und der Behandlungskörper dadurch kraftschonend vom Gliedabschnitt abnehmbar ist.

Die Steuerungseinrichtung weist einen vorzugsweise integrierten Schalter zur Aktivierung und/oder Deaktivierung der Bestrahlungseinrichtung auf, vorzugsweise wobei der Schalter an einer äußeren Ringfläche des Behandlungskörpers angeordnet ist, insbesondere wobei der Schalter gegenüberliegend zur Aussparung oder zu den Befestigungslaschen angeordnet ist. Dies ermöglicht eine komfortable und/oder intuitive Bedienung des Schalters, da der Nutzer vorzugsweise durch Ertasten der Unterbrechung bzw. der Befestigungslaschen den gegenüberliegend hierzu angeordneten Schalter erfühlen bzw. ertasten kann. Alternativ oder zusätzlich kann der Schalter auch durch taktile Hervorhebung, beispielsweise durch eine Aufwölbung, Einkerbung, Aufrauhung oder eine sonstige Oberflächenmodifizierung gegenüber angrenzenden Oberflächen hervorgehoben sein, um die Betätigung entsprechend intuitiver zu gestalten bzw. zu vereinfachen.

Was den inneren Aufbau des Behandlungskörpers anbelangt, so ist vorzugsweise ein Gehäuse oder wenigstens ein Gehäuseteil bzw. Gehäuseabschnitt aus einem lichtundurchlässigen Material hergestellt und/oder weist eine lichtundurchlässige Oberfläche auf und/oder ist in dem Gehäuse bzw. Gehäuseteil wenigstens eine Lichtöffnung für eine Lichtabgabe radial nach innen und/oder in Richtung zur Mitte des Behandlungskörpers vorgesehen. Vorzugsweise weist der Behandlungskörper ein mehrteiliges Gehäuse auf. Beispielsweise weist der Behandlungskörper ein aus zwei Halbschalen gebildetes Gehäuse auf, in welchem vorzugsweise sämtliche Elektronikkomponenten, zumindest jedoch die Bestrahlungseinrichtung und/oder die Stromquelle und/oder die Steuerschaltung, integriert bzw. aufgenommen, insbesondere eingekapselt, sind.

Vorzugsweise besteht der Behandlungskörper bzw. das Gehäuse des Behandlungskörpers zumindest teilweise aus einem elastischen Material, insbesondere um eine flexible Erweiterung der Öffnungsweite des Behandlungskörpers sicherzustellen. Hierzu kann das Gehäuse an sich aus einem elastischen Material bestehen. Alternativ können jedoch auch lediglich Gehäuseabschnitte bzw. wenigstens ein Gehäuseteil elastisch ausgebildet sein, beispielsweise zur Ausbildung eines Scharniergelenks, welches zwei schwenkbar zueinander angebundene Gehäuseteile des Behandlungskörpers miteinander verbindet. Diese gelenkig miteinander verbundenen Gehäuseteile können dann auch starr ausgebildet sein. Auch der Einsatz von Federelementen bzw. Federabschnitten zur Ausbildung gelenkiger Abschnitte bzw. Scharnierabschnitte sind zur individuellen Einstellung der Öffnungsweite möglich.

Wenigstens ein Gehäuse oder Gehäuseteil des Behandlungskörpers weist vorzugsweise zumindest abschnittsweise eine Ummantelung und/oder Beschichtung aus einem insbesondere elastischen Kunststoffmaterial auf, insbesondere eine Silikon-, Kautschuk-, Latex- und/oder Polyurethanummantelung und/oder -beschichtung.

Besonders bevorzugt ist eine Ummantelung und/oder Beschichtung unter Aussparung der Lichtöffnung bzw. der Lichtaustrittsfläche unterbrechungsfrei und/oder in sich geschlossen am Behandlungskörper vorgesehen. Dadurch wird eine im Wesentlichen durchgehende und flüssigkeitsdichte Außenschicht gebildet, wobei die vollflächige, insbesondere elastische Beschichtung eine besonders hohe Anschmiegung an den Penisumfang und somit eine besonders effiziente Therapie bzw. Bestrahlung ermöglicht.

Besonders bevorzugt ist die Ummantelung und/oder Beschichtung mit einer Lichtaustrittsfläche des Lichtmittels und/oder einer Oberfläche des Verschlusselements ausgefluchtet und/oder glatt bzw. unterbrechungsfrei in diese übergehend ausgebildet. Dadurch erfolgt eine insbesondere licht- und/oder flüssigkeitsabdichtende Anbindung zwischen der Lichtaustrittsfläche bzw. dem Verschlussmittel einerseits und der angrenzenden Ummantelung andererseits, insbesondere so dass eine besonders gerichtete Bestrahlung radial nach innen ermöglicht wird, wobei die lichtundurchlässige Ummantelung eine scharfe Abgrenzung zu diesem Lichtaustritt ermöglicht bzw. begünstigt.

Eine derartige Realisierung ist jedoch nicht zwingend. So kann es ebenfalls vorteilhaft sein, wenn die Ummantelung und/oder Beschichtung zumindest angrenzend bzw. im Umgebungsbereich des jeweiligen Lichtmittels bzw. des jeweiligen Verschlusselements lichtdurchlässig bzw. transparent ist. Dadurch kann eine Abstrahlung über das Lichtmittel in das angrenzende transparente Material der Ummantelung und/oder Beschichtung erfolgen, so dass eine Zerstreuung des durch das Lichtmittel erzeugten Lichts über die angrenzende Ummantelung bzw. Beschichtung erfolgt und somit auch in diesem Bereich eine gewisse Bestrahlung des umschlossenen Glieds erfolgt. Dadurch wird gewissermaßen die Bestrahlungsfläche aufgeweitet und somit die Bestrahlung insgesamt noch vollflächiger bzw. intensiver ausgebildet.

Die Oberfläche des Behandlungskörpers kann mechanisch und/oder chemisch modifiziert sein und/oder es kann eine Oberflächenbeschichtung vorgesehen sein, um das Anhaften von wenigstens einem insbesondere wasserbasierten Photosensibilisator zu verbessern. Als Haftmittel kann beispielsweise Cellulose dienen. Wird ein Photosensibilisator auf den Behandlungskörper aufgebracht, kann dieser gefolgt von einer Beleuchtung eine Lichttherapie unterstützen.

Um die therapeutische bzw. erektile Wirkung zu intensivieren bzw. zu unterstützen kann der Penisring auch einen Vibrationsmechanismus bzw. eine Schwingungserzeugungseinrichtung aufweisen, wie er beispielsweise in der DE 20 2016 000 683 U1 gezeigt ist.

Bevorzugte Ausführungsformen des erfindungsgemäßen Penisrings werden nachfolgend anhand der Zeichnung beispielhaft beschrieben, wobei die Erfindung nicht auf die beschriebenen Ausführungsformen beschränkt ist. In der Zeichnung zeigen
- Fig. 1: eine perspektivische Ansicht eines erfindungsgemäßen Penisrings gemäß einer ersten Ausführungsform, jedoch mit nicht in zugeordnete Lichtöffnungen eingesetzte Lichtmittel,
- Fig. 2: der erfindungsgemäße Penisring gemäß der ersten Ausführungsform im aktivierten Zustand bzw. mit aktivierten Lichtmitteln,
- Fig. 3: eine perspektivische Ansicht des erfindungsgemäßen Penisrings gemäß einer zweiten Ausführungsform und
- Fig. 4: eine weitere perspektivische Ansicht des erfindungsgemäßen Penisrings der zweiten Ausführungsform.

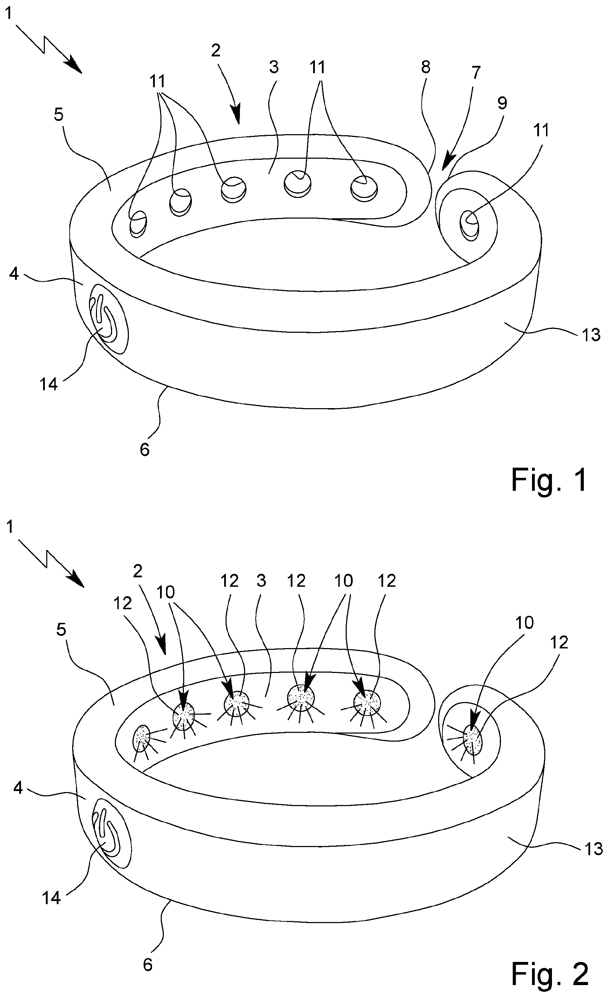

In den Fign. 1 und 2 ist ein Penisring 1 mit einem ringförmigen Behandlungskörper 2 zum Umgreifen des Penis vorgesehen.

Dementsprechend weist der Behandlungskörper 2 eine innere Ringfläche 3 sowie eine äußere Ringfläche 4 auf. Dabei ist die innere Ringfläche 3 im Anwendungszustand dem Penis zugewandt, wohingegen die äußere Ringfläche 4 dementsprechend vom Penis abgewandt ist. Die innere Ringfläche 3 bildet eine vorzugsweise durchgehende Kontakt- bzw. Behandlungsfläche mit dem Penis aus.

Die innere Ringfläche 3 sowie die äußere Ringfläche 4 sind über eine erste Stirnfläche 5 sowie eine, in der Perspektivansicht abgedeckte, zweite Stirnfläche 6 miteinander verbunden.

Die Stirnflächen 5, 6 können eben bzw. flach ausgebildet sein. Alternativ können die Stirnflächen 5, 6 jedoch auch gewölbt sein, insbesondere um einen gewölbten bzw. kantenfreien Übergang zur inneren Ringfläche 3 und/oder zur äußeren Ringfläche 4 zu ermöglichen.

Der Behandlungskörper 2 ist vorzugsweise zur Anpassung und/oder Erweiterung der Öffnungsweite zumindest teilweise elastisch ausgebildet, insbesondere um die Anwendung über einen weiten Bereich von Penisgrößen bzw. Penisumfängen sicherzustellen.

Beim dargestellten Ausführungsbeispiel weist der Behandlungskörpers 2 eine sich über die gesamte axiale Höhe des Behandlungskörpers 2 erstreckende Unterbrechung 7 auf. Die Unterbrechung 7 ist dabei durch zwei aufeinander zuweisende, vorzugsweise abgerundete, freie Enden 8, 9 des Behandlungskörpers 2 seitlich begrenzt. Durch Ergreifen des Behandlungskörpers 2, beispielsweise im Bereich der freien Enden 8, 9, ist eine flexible Aufweitung des Behandlungskörpers 2 im Rahmen des Umgreifens bzw. Anlegens des Behandlungskörpers 2 möglich. Vorzugsweise erfolgt nach dem Anlegen eine elastische Rückstellung, so dass der Behandlungskörper 2 unter Einwirkung einer gewissen Einschnürung bzw. Druckausübung umfangsseitig am Penisabschnitt zur Anlage kommt, und zwar vorzugsweise vollflächig mit der inneren Ringfläche 3.

Der erfindungsgemäße Penisring 1 bzw. Behandlungskörper 2 ist, zusätzlich zum vorzugsweise festen Umgreifen des Penis, zur gezielten Bestrahlung desselben ausgebildet, insbesondere zur (weiteren) erektilen Stimulation und/oder zur Erhöhung der Spermienaktivität, insbesondere zu Zwecken der Potenzsteigerung.

Hierzu ist im Behandlungskörper 2 eine Bestrahlungseinrichtung mit einer Mehrzahl von Leuchtmitteln 10 integriert, wie in Fig. 2 ersichtlich.

Dabei ist die Bestrahlungseinrichtung bzw. sind die Leuchtmittel 10 zur Emission von blauem und/oder blauviolettem Licht im sichtbaren Längenwellenbereich, insbesondere mit einer Wellenlänge zwischen 400 nm und 470 nm, ausgebildet bzw. konzeptioniert. Wie anhand von Fig. 2 ersichtlich, sind die Leuchtmittel 10 auf der inneren Ringfläche 3 des Behandlungskörpers 2 angeordnet und ermöglichen dadurch eine radial nach innen und/oder in das Zentrum des Behandlungskörpers 2 gerichtete Bestrahlung.

Insbesondere sind die Leuchtmittel 10 konfiguriert zur Emission von Licht mit einer Wellenlänge zwischen 400 Nm und 460 Nm, vorzugsweise zwischen 410 Nm und 450 Nm. Die durch die Leuchtmittel 10 eingebrachte Bestrahlungsstärke liegt dabei zwischen 0,1 mW/cm² und 2,0 mW/cm², vorzugsweise zwischen 0,5 mW/cm² und 1,5 mW/cm².

Zur Steuerung der Bestrahlungseinrichtung bzw. der Leuchtmittel 10 weist der Behandlungskörper 2 eine vorzugsweise integrierte Steuerschaltung zum automatischen Abschalten der Bestrahlung bei Erreichen einer Bestrahlungsenergie von weniger als 37 J, vorzugsweise von weniger als 5,0 J, weiter vorzugsweise von weniger als 2,0 J, besonders bevorzugt von 0,5 J bis 1,5 J und/oder nach einer Bestrahlungsdauer von kleiner oder gleich 60 min, vorzugsweise von kleiner oder gleich 30 min, weiter vorzugsweise von kleiner oder gleich 20 min.

Wie anhand von Fig. 2 ersichtlich, weist der Behandlungskörper 2 mehrere in Umfangsrichtung gleichverteilt angeordnete Leuchtmittel 10 auf, insbesondere wobei die Leuchtmittel 10 auf mittlerer Höhe auf der inneren Ringfläche 3 in gleichverteilten Abständen zueinander angeordnet sind. Dabei sind die Leuchtmittel 10 vorzugsweise in gleichen Versatzwinkeln in Umfangsrichtung zueinander versetzt, wobei für den Versatzwinkel die Vorschrift 360°/n, mit n als Anzahl der Leuchtmittel 10, gilt.

Die Leuchtmittel 10 sind über die gesamte innere Ringfläche 3 des Behandlungskörpers 2 angeordnet, um eine Behandlung des Gliedes über den gesamten Umfang in gleichmäßiger Weise zu ermöglichen.

Was die Anbindung der Leuchtmittel 10 anbelangt, so ist vorzugsweise für jedes Leuchtmittel 10 eine separate Lichtöffnung 11 vorgesehen, insbesondere zur passgenauen Aufnahme des Leuchtmittels 10. Das Leuchtmittel 10 ist dabei insbesondere passgenau in die zugeordnete Lichtöffnung 11 einsetzbar und wird dann sicher, insbesondere abdichtend, in dieser aufgenommen bzw. gehalten.

Beim dargestellten und bevorzugten Ausführungsbeispiel sind runde bzw. kreisförmige Lichtöffnungen 11 vorgesehen, in welchen die entsprechenden Lechtmittel 10 aufgenommen sind. Dabei beträgt das Verhältnis der Höhe bzw. axialen Erstreckung des Behandlungskörpers 2 einerseits zu den vorzugsweise gleichen Durchmessern der Lichtöffnungen 11 andererseits vorzugsweise wenigstens 30 % und höchstens 60 %, insbesondere bezogen auf die Höhe bzw. axiale Erstreckung der inneren Ringfläche 3.

Die Lichtöffnungen 11 und/oder die mit den Leuchtmitteln 10 einhergehenden Lichtaustrittsflächen erstrecken sich über einen Bereich von 25 bis 90 % des Umfangs der inneren Ringfläche 3. Dabei nehmen die Lichtöffnungen 11 bzw. die Lichtaustrittsflächen der Lichtmittel 11 einen Flächenanteil zwischen 20 und 80 % der inneren Ringfläche 3 ein. Die restliche, nicht auf die Lichtöffnungen 11 bzw. die Lichtaustrittsflächen entfallenen Restflächen der inneren Ringfläche 3 bilden eine vorzugsweise lichtundurchlässige Kontaktfläche mit dem Penisabschnitt im Anwendungszustand aus, jedoch mit dem Unterschied, dass in diesen Flächenabschnitten keine oder eine reduzierte Emission von Licht stattfindet. Die gesamte innere Ringfläche 3 ist dementsprechend durch die Lichtöffnungen 11 bzw. Lichtaustrittsflächen einerseits sowie die nicht mit der Emission von Licht in Verbindung stehenden Flächenanteilen bzw. Kontaktflächen andererseits gebildet.

Hier sind jedoch auch andere technische Lösungen möglich, beispielsweise eine lichtdurchlässige bzw. transparente Ausbildung der Kontaktfläche, insbesondere im Umfangsbereich der zugeordneten Lichtöffnungen 11, um eine Lichtstreuung auch im Umgebungsbereich der Lichtöffnungen 11 zu realisieren bzw. zu unterstützen.

Beim dargestellten und bevorzugten Ausführungsbeispiel ist jede Lichtöffnung 11 mit einem scheibenartigen Verschlusselement 12 verschlossen. Insbesondere sind die Verschlusselemente 12 zur passgenauen Anwendung in die zugeordnete Lichtöffnungen 11 ausgebildet bzw. komplementär zu dieser dimensioniert. Dies gestattet ein zuverlässiges, insbesondere abgedichtetes, Verschließen des integrierten Leuchtmittels 11 mittels der darauf aufgesetzten Verschlusselemente 12.

Die Verschlusselemente 12 weisen dabei vorzugsweise eine satinierte und/oder mattierte Oberfläche auf und/oder bestehen aus einem transparenten bzw. lichtdurchlässigen Material, insbesondere aus Kunststoff, weiter insbesondere aus ABS. Alternativ oder zusätzlich können die Verschlusselemente 12 als Sammel- oder Zerstreuungslinse ausgebildet sein, insbesondere um eine fokussierte Abstrahlung radial nach innen bzw. in Richtung des Zentrums des Behandlungskörpers 2 zu ermöglichen.

Die Anbindung bzw. Integration des Verschlusselements 12 wird vorzugsweise derart an der inneren Ringfläche 3 gewählt, dass das Verschlusselement 12 oberseitig mit einer angrenzenden Oberfläche der inneren Ringfläche 3 ausgefluchtet ist und/oder glatt bzw. unterbrechungsfrei in diese übergeht. Dies ist einer komfortablen Anwendung zuträglich, ohne dass die innere Ringfläche 3 mit spürbaren bzw. störenden Übergängen in Kontakt mit der zu bestrahlenden (Haut-)Fläche tritt.

In diesem Zusammenhang ist im Sinne des besonders bevorzugten Ausführungsbeispiels eine Ummantelung 13 vorgesehen, durch welche die Verschlusselemente 12 umgrenzt bzw. umschlossen sind. Dabei besteht die Ummantelung und/oder Beschichtung aus einem insbesondere elastischen Kunststoffmaterial, insbesondere aus einer vorzugsweise tranparenten Silikon-, Kautschuk-, Latex- und/oder Polyurethanummantelung und/oder -beschichtung. Es versteht sich, dass die Ummantelung unter Aussparung der Lichtöffnungen 11 unterbrechungsfrei und/oder in sich geschlossen am Behandlungskörper 2 vorgesehen ist, vorzugsweise derart, dass die Ummantelung 13 mit den Verschlusselementen 12 ausgefluchtet ist bzw. glatt und/oder unterbrechungsfrei in diese übergeht.

Besonders bevorzugt ist die Ummantelung 13 aus einem transparenten Material, insbesondere aus einem transparenten Silikon, gebildet, in welches die Lichtmittel 10 und/oder die Verschlusselemente 12 eingebracht, vorzugsweise eingebettet, beispielsweise eingeklebt und/oder eingeschweißt, sind.

Zur Aktivierung und/oder Deaktivierung der Beleuchtungseinrichtung bzw. der Leuchtmittel 10 weist die vorzugsweise integrierte Steuerungseinrichtung einen vorzugsweise integrierten Schalter 14 auf, vorzugsweise wobei der Schalter 14 an der äußeren Ringfläche 4 des Behandlungskörpers 2 angeordnet ist, beim Darstellungsbeispiel gegenüberliegend zur Aussparung 7 angeordnet ist. Dabei kann der Schalter 14 durch die Ummantelung 13 gebildet sein, beispielsweise in Form einer Wölbung oder eine sonstige Erhöhung oder Vertiefung, um dem Anwender einen taktilen Anhaltspunkt zur Lage des Schalters 14 zu übermitteln.

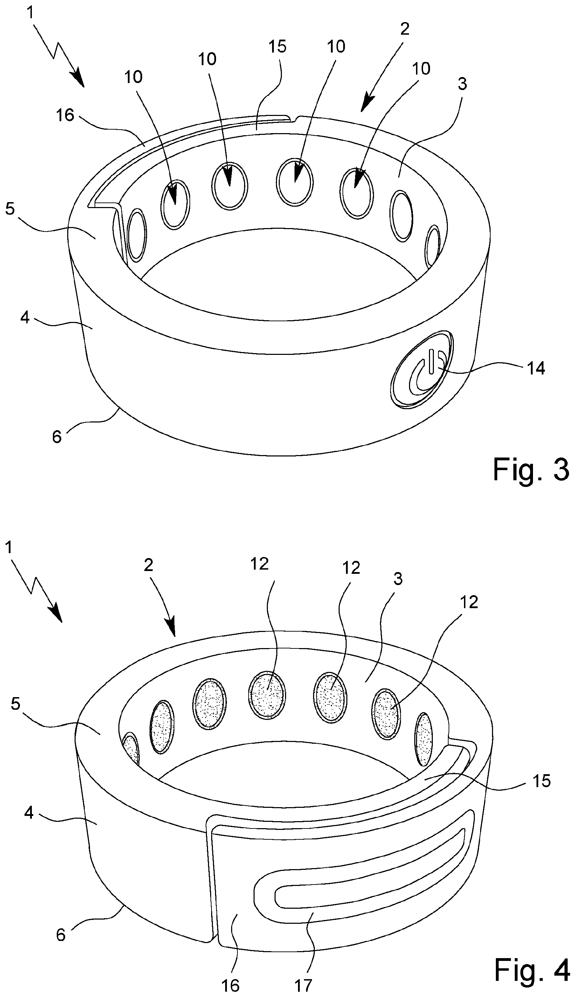

Nachfolgend wird anhand von Fign. 3 und 4 eine weitere, alternative bzw. zweite Ausführungsform des erfindungsgemäßen Penisrings 1 beschrieben. Merkmale, die in Bezug auf die erste Ausführungsform erörtert worden sind, gelten dabei gleichermaßen auch für diese, zweite Ausführungsform, auch wenn auf eine wiederholte Beschreibung verzichtet wird.

Die nachfolgend erörterte, zweite Ausführungsform unterscheidet sich von der ersten Ausführungsform maßgeblich in der Realisierung von zwei einander überlappenden, gekrümmten Befestigungslaschen 15, 16. Dabei sind eine innere Befestigungslasche 15, welche der inneren Ringfläche 3 zugeordnet ist, sowie eine äußere Befestigungslasche 16, welche der äußeren Ringfläche 4 zugeordnet ist, vorgesehen. Zur Ausbildung der Überlappung greift die äußere Befestigungslasche 16 in eine gegenüberliegend zur inneren Befestigungslasche 15 vorgesehene Aussparung ein, insbesondere derart, dass die äußere Befestigungslasche 16 ohne radialen Überstand in die angrenzende äußere Ringfläche 4 des Behandlungskörpers 2 übergeht.

Der Schalter 14 ist vorzugsweise gegenüberliegend zu den Befestigungslaschen 15, 16 angeordnet.

Beim dargestellten und bevorzugten Ausführungsbeispiel sind die Befestigungslaschen 15, 16 lösbar miteinander verbunden, und zwar über magnetische Kopplung. Hierzu weist die innere Befestigungslasche und/oder die äußere Befestigungslasche 16 mindestens einen magnetischen Abschnitt 17 auf.

Ein weiterer, nicht explizit dargestellter Aspekt der vorliegenden Erfindung, betrifft ebenfalls ein Kit, bestehend aus einen Aufbewahrungsbehälter, insbesondere einer Aufbewahrungsbox, beispielsweise einer Schatulle, und dem erfindungsgemäßen Penisring 1, der in dem Aufbewahrungsbehälter aufgenommen ist. Der Aufbewahrungsbehälter ist dabei neben der Aufbewahrung des erfindungsgemäßen Penisrings 1 gleichzeitig zum Aufladen des Akkumulators bzw. Speichers des Penisrings 1 ausgebildet, insbesondere mittels Induktion. Hierzu weist ein Begrenzungsabschnitt des Aufbewahrungsbehälters, insbesondere der Boden, eine Ladefläche auf, über die der in dem Aufbewahrungsbehälter aufgenommene Penisring 1 mittels Induktion entsprechend aufladbar ist. Zur Energieübertragung über die Ladefläche weist der Aufnahmebehälter dabei einen Energieanschluss, insbesondere in Form eines USB-Anschlusses, auf, über den eine Stromquelle anschließbar ist, die letztlich zur Energieübertragung über die Ladefläche an den Penisring 1 bzw. den Akkumulator des Penisrings 1 ausgebildet ist.

Hierdurch wird ein benutzerfreundliches, kompaktes Kit zum flexiblen Einsatz des Penisrings 1 zur Verfügung gestellt, der darüber hinaus mit einer anschaulichen bzw. verkaufsfördernden Ausstrahlung einhergeht.

### Bezugszeichenliste:

- 1: Penisring
- 2: Behandlungskörper
- 3: innere Ringfläche
- 4: äußere Ringfläche
- 5: Stirnfläche
- 6: Stirnfläche
- 7: Unterbrechung
- 8: freies Ende
- 9: freies Ende

- 10: Leuchtmittel
- 11: Lichtöffnung
- 12: Verschlusselement
- 13: Ummantelung
- 14: Schalter
- 15: Befestigungslasche
- 16: Befestigungslasche
- 17: Magnetabschnitt

## Patentansprüche

1. Penisring (1), insbesondere zur sexuellen Stimulation und/oder zur Förderung der Erektion des Mannes, besonders bevorzugt zur Erhöhung der Spermienaktivität, mit einem ringförmigen Behandlungskörper (2) zum Umgreifen des Penis, mit einer in dem Behandlungskörper (2) integrierten Bestrahlungseinrichtung mit wenigstens einem Leuchtmittel (10), insbesondere LED-Leuchtmittel, mit einer integrierten Stromquelle zur Stromversorgung der Bestrahlungseinrichtung und mit einer Steuerschaltung zur Steuerung der Bestrahlungseinrichtung, wobei die Bestrahlungseinrichtung konfiguriert ist zur Emission von blauem und/oder blauviolettem Licht im sichtbaren Wellenlängenbereich mit einer Wellenlänge zwischen 410 nm und 450 nm, wobei die Bestrahlungseinrichtung konfiguriert ist zur Beleuchtung mit einer Bestrahlungsstärke zwischen 0,1 mW/cm² und 2,0 mW/cm² und wobei die Steuerschaltung zum automatischen Abschalten der Bestrahlung bei Erreichen einer Bestrahlungsenergie von weniger als 5,0 J und/oder nach einer Bestrahlungsdauer von kleiner oder gleich 60 min ausgebildet ist.

2. Penisring nach Anspruch 1, wobei die Bestrahlungseinrichtung konfiguriert ist zur Beleuchtung mit einer Bestrahlungsstärke zwischen 0,5 mW/cm² und 1,5 mW/cm².

3. Penisring nach Anspruch 1 oder 2, wobei die Steuerschaltung zum automatischen Abschalten der Bestrahlung bei Erreichen einer Bestrahlungsenergie von weniger als 2,0 J, besonders bevorzugt von 0,5 J bis 1,5 J, und/oder nach einer Bestrahlungsdauer von kleiner oder gleich 30 min, weiter vorzugsweise von kleiner oder gleich 20 min, ausgebildet ist.

4. Penisring nach einem der vorhergehenden Ansprüche, wobei eine Anordnung des Leuchtmittels (10) und/oder ein Abstrahlwinkel des Leuchtmittels (10) vorgesehen ist, die bzw. der eine radial nach innen gerichtete und/oder in das Zentrum des Behandlungskörpers (2) gerichtete Bestrahlung ermöglicht.

5. Penisring nach einem der vorhergehenden Ansprüche, wobei die Bestrahlungseinrichtung mehrere Leuchtmittel (10) aufweist, vorzugsweise wobei die Leuchtmittel (10) in Umfangsrichtung, vorzugsweise gleichverteilt, entlang einer inneren Ringfläche des Behandlungskörpers angeordnet sind und/oder wobei die Leuchtmittel (10) in Umfangsrichtung, vorzugsweise gleichverteilt, über die gesamte innere Ringfläche (3) des Behandlungskörpers (2) angeordnet sind.

6. Penisring nach einem der vorhergehenden Ansprüche, wobei der Behandlungskörper (2) vorzugsweise auf der inneren Ringfläche (3) wenigstens eine Lichtöffnung (11) zur insbesondere passgenauen Aufnahme des wenigstens einen Leuchtmittels (10) aufweist, insbesondere wobei sich die Lichtöffnung (11) über einen Bereich von 25 bis 75 %, vorzugsweise über einen Bereich von 40 bis 60 %, bezogen auf die axiale Höhe des Behandlungskörpers (2), vorzugsweise der inneren Ringfläche (3), erstreckt und/oder wobei sich die Lichtöffnung (11) und/oder eine Lichtaustrittsfläche des Lichtmittels (10) über einen Bereich von 25 bis 90 %, vorzugsweise von 40 bis 60 %, über den Umfang des Behandlungskörpers (2), vorzugsweise der inneren Ringfläche (3), erstreckt und/oder wobei die Lichtöffnung(en) (11) von einer in sich geschlossenen, integralen Oberfläche bzw. Ummantelung (13) des Behandlungskörpers (2) umgrenzt und/oder umschlossen ist bzw. sind.

7. Penisring nach Anspruch 6, wobei die Fläche der Lichtöffnung (11) und/oder Lichtaustrittsfläche zwischen 20 und 80 %, vorzugsweise zwischen 40 und 60 %, der inneren Ringfläche (3) des Behandlungskörpers (2) beträgt bzw. einnimmt und/oder dass der Behandlungskörper (2), vorzugsweise auf der inneren Ringfläche (3), mehrere Lichtöffnungen (11) aufweist, wobei, vorzugsweise jedem Leuchtmittel (10) eine separate Lichtöffnung (11) zugeordnet ist, vorzugsweise wobei die Lichtöffnungen (11) über den gesamten Umfang des Behandlungskörpers (2), vorzugsweise der inneren Ringfläche (3) verteilt sind, vorzugsweise gleichverteilt sind.

8. Penisring nach Anspruch 7, wobei sich die Lichtöffnungen (11) in Summe über einen Anteil von 30 bis 80 %, vorzugsweise von 40 bis 70 %, insbesondere von 50 bis 60 %, entlang des Umfangs des Behandlungskörpers (2), vorzugsweise der inneren Ringfläche (3), erstrecken und/oder dass die Fläche aller Lichtöffnungen (11) zwischen 20 und 80 %, vorzugsweise zwischen 40 und 60 %, einer Ringfläche (3, 4) des Behandlungskörpers (2), vorzugsweise der inneren Ringfläche (3) des Behandlungskörpers (2), beträgt bzw. einnimmt.

9. Penisring nach einem der Ansprüche 6 bis 8, wobei die Lichtöffnung (11) und/oder eine Lichtaustrittsfläche des Leuchtmittels (10) langgestreckt und/oder bandförmig ausgebildet ist und/oder dass die Lichtöffnung (11) rund, insbesondere kreisförmig, oval oder punktförmig ausgebildet ist und/oder dass vorzugsweise jede Lichtöffnung (11) mit einem insbesondere scheibenartigen Verschlusselement (10) aus einem lichtdurchlässigen Material verschlossen ist, insbesondere wobei vorzugsweise jedes Verschlusselement (12) eine satinierte und/oder mattierte Oberfläche aufweist und/oder wobei vorzugsweise jedes Verschlusselement (12) aus einem transparenten und/oder lichtdurchlässigen Material, insbesondere aus Kunststoff, weiter insbesondere aus ABS, besteht und/oder ein solches Material aufweist und/oder wobei dass das Verschlusselement (12) als Sammellinse oder Zerstreuungslinse ausgebildet ist.

10. Penisring nach einem der vorhergehenden Ansprüche, wobei eine Lichtaustrittsfläche des Leuchtmittels (10), insbesondere eines Verschlusselements (12), mit einer angrenzenden Oberfläche, insbesondere einer Ummantelung, des Behandlungskörpers (2) ausgefluchtet ist und/oder glatt bzw. unterbrechungsfrei in diese übergeht und/oder dass die Stromquelle als austauschbarer und/oder wieder aufladbarer Akkumulator für elektrische Energie vorgesehen ist, insbesondere eine Knopfzelle, und/oder dass eine vorzugsweise integrierte Einrichtung zum kabellosen induktiven Wiederaufladen des Akkumulators vorgesehen ist.

11. Penisring nach einem der vorhergehenden Ansprüche, wobei der Behandlungskörper (2) zumindest teilweise elastisch ausgebildet ist und/oder flexibel in seiner Öffnungsweite, vorzugsweise elastisch flexibel, einstellbar und/oder aufweitbar ausgebildet ist und/oder dass der Behandlungskörper (2) eine sich vorzugsweise über die gesamte axiale Höhe des Behandlungskörpers (2) erstreckende Unterbrechung (7) aufweist.

12. Penisring nach einem der vorhergehenden Ansprüche, wobei der Behandlungskörper (2) zwei einander überlappende, gekrümmte Befestigungslaschen (15, 16) aufweist, insbesondere wobei die Befestigungslaschen (15, 16) lösbar, vorzugsweise form- und/oder reibschlüssig, insbesondere magnetisch, miteinander verbunden sind und/oder wobei der Behandlungskörper (2) durch Ergreifen der freien Enden oder der Befestigungslaschen (15, 16) in seiner Öffnungsweite vorzugsweise elastisch verstellbar und/oder aufweitbar ist.

13. Penisring nach einem der Ansprüche 11 oder 12, wobei die Steuerungseinrichtung einen vorzugsweise integrierten Schalter zur Aktivierung und/oder Deaktivierung der Bestrahlungsvorrichtung aufweist, vorzugsweise wobei der Schalter (14) an einer äußeren Ringfläche (4) des Behandlungskörpers (2) angeordnet ist, insbesondere wobei der Schalter (14) gegenüberliegend zur Unterbrechung und/oder zu den Befestigungslaschen (15, 16) angeordnet ist und/oder dass ein Gehäuse oder wenigstens ein Gehäuseteil des Behandlungskörpers (2) aus einem lichtundurchlässigen Material besteht und/oder eine lichtundurchlässige Oberfläche aufweist und/oder dass in dem Gehäuse bzw. Gehäuseteil wenigstens eine Lichtöffnung vorgesehen ist für eine Lichtabgabe radial nach innen und/oder in Richtung zur Mitte des Behandlungskörpers (2).

14. Penisring nach einem der vorhergehenden Ansprüche, wobei wenigstens ein Gehäuse oder ein Gehäuseteil zumindest abschnittsweise eine Ummantelung (13) und/oder Beschichtung aus einem insbesondere elastischen Kunststoffmaterial aufweist, insbesondere eine Silikon-, Kautschuk-, Latex- und/oder Polyurethanummantelung und/oder -beschichtung, vorzugsweise wobei die Ummantelung (13) und/oder Beschichtung unter Aussparung der Lichtöffnung (11) bzw. Lichtöffnungen (11) unterbrechungsfrei und/oder in sich geschlossen am Behandlungskörper (2) vorgesehen ist.

15. Penisring nach Anspruch 14, wobei die Ummantelung (13) und/oder Beschichtung mit einer Lichtaustrittsfläche des Lichtmittels (10) und/oder einer Oberfläche des Verschlusselements (12) ausgefluchtet ist und/oder glatt bzw. unterbrechungsfrei in diese übergeht und/oder dass die Oberfläche, vorzugsweise die innere Ringfläche (3), des Behandlungskörpers (2) mechanisch und/oder chemisch modifiziert ist und/oder die Ummantelung (13) und/oder Beschichtung aufweist, um das Anhaften von wenigstens einem insbesondere wasserbasierten Photosensibilisator zu verbessern.

## Claims

1. Penis ring (1), in particular for sexual stimulation and/or for promoting male erection, particularly preferred for increasing sperm activity, with a ring-shaped treatment body (2) for encircling the penis, with an irradiation device integrated in the treatment body (2) with at least one light source (10), in particular LED light source, with an integrated power source for supplying power to the irradiation device and with a control circuit for controlling the irradiation device, wherein the irradiation device is configured to emit blue and/or blue-violet light in the visible wavelength range with a wavelength between 410 nm and 450 nm, wherein the irradiation device is configured for illumination with an irradiance between 0.1 mW/cm² and 2.0 mW/cm² and wherein the control circuit is designed to automatically switch off the irradiation when an irradiation energy of less than 5.0 J is reached and/or after an irradiation duration of less than or equal to 60 min.

2. Penis ring according to claim 1, wherein the irradiation device is configured for illumination with an irradiance between 0.5 mW/cm² and 1.5 mW/cm².

3. Penis ring according to claim 1 or 2, wherein the control circuit is designed to automatically switch off the irradiation when an irradiation energy of less than 2.0 J, particularly preferably from 0.5 J to 1.5 J, is reached and/or after an irradiation duration of less than or equal to 30 minutes, more preferably less than or equal to 20 minutes.

4. Penis ring according to one of the preceding claims, wherein an arrangement of the light source (10) and/or a beam angle of the light source (10) is provided which enables irradiation directed radially inwards and/or towards the centre of the treatment body (2).

5. Penis ring according to one of the preceding claims, wherein the irradiation device has several light sources (10), preferably wherein the light sources (10) are arranged in the circumferential direction, preferably evenly distributed, along an inner ring surface of the treatment body and/or wherein the light sources (10) are arranged in the circumferential direction, preferably evenly distributed, over the entire inner ring surface (3) of the treatment body (2).

6. Penis ring according to one of the preceding claims, wherein the treatment body (2) preferably has at least one light opening (11) on the inner ring surface (3) for the particularly precise accommodation of the at least one light source (10), in particular wherein the light opening (11) extends over an area of 25 to 75%, preferably over an area of 40 to 60%, relative to the axial height of the treatment body (2), preferably the inner ring surface (3), and/or wherein the light opening (11) and/or a light exit surface of the light source (10) extends over an area of 25 to 90%, preferably 40 to 60%, over the circumference of the treatment body (2), preferably the inner ring surface (3), and/or wherein the light opening(s) (11) is/are bounded and/or enclosed by a self-contained, integral surface or sheathing (13) of the treatment body (2).

7. Penis ring according to claim 6, wherein the area of the light opening (11) and/or light exit area is between 20 and 80%, preferably between 40 and 60%, of the inner ring surface (3) of the treatment body (2) and/or the treatment body (2) preferably on the inner ring surface (3), has several light openings (11), wherein, preferably, a separate light opening (11) is assigned to each light source (10), preferably wherein the light openings (11) are distributed over the entire circumference of the treatment body (2), preferably the inner ring surface (3), preferably evenly distributed.

8. Penis ring according to claim 7, wherein the light openings (11) extend in total over a proportion of 30 to 80%, preferably 40 to 70%, in particular 50 to 60%, along the circumference of the treatment body (2), preferably the inner ring surface (3), and/or that the area of all light openings (11) is between 20 and 80%, preferably between 40 and 60%, of a ring surface (3, 4) of the treatment body (2), preferably the inner ring surface (3) of the treatment body (2).

9. Penis ring according to one of claims 6 to 8, wherein the light opening (11) and/or a light-emitting surface of the light source (10) is elongated and/or strip-shaped and/or the light opening (11) is round, in particular circular, oval or point-shaped, and/or that preferably each light opening (11) is closed with a disc-like closure element (10) made of a light-transmissive material, in particular wherein preferably each closure element (12) has a satinised and/or matt surface and/or preferably each closure element (12) consists of a transparent and/or translucent material, in particular plastic, in particular ABS, and/or has such a material and/or the closure element (12) is designed as a converging lens or diverging lens.

10. Penis ring according to one of the preceding claims, wherein a light-emitting surface of the light source (10), in particular a closure element (12), is aligned with an adjacent surface, in particular a casing, of the treatment body (2) and/or merges smoothly and without interruption into it, and/or that the power source is provided as a replaceable and/or rechargeable accumulator for electrical energy, in particular a button cell, and/or that a preferably integrated device for wireless inductive recharging of the accumulator is provided.

11. Penis ring according to one of the preceding claims, wherein the treatment body (2) is at least partially elastic and/or flexible in its opening width, preferably elastically flexible, adjustable and/or expandable, and/or that the treatment body (2) has an interruption (7) extending preferably over the entire axial height of the treatment body (2).

12. Penis ring according to one of the preceding claims, wherein the treatment body (2) has two overlapping, curved fastening tabs (15, 16), in particular wherein the fastening tabs (15, 16) are detachably connected to one another, preferably by form and/or friction locking, in particular magnetically, and/or wherein the treatment body (2) is preferably elastically adjustable and/or expandable in its opening width by grasping the free ends or the fastening tabs (15, 16).

13. Penis ring according to one of claims 11 or 12, wherein the control device has a preferably integrated switch for activating and/or deactivating the irradiation device, preferably wherein the switch (14) is arranged on an outer ring surface (4) of the treatment body (2), in particular wherein the switch (14) is arranged opposite the interruption and/or the fastening tabs (15, 16) and/or that a housing or at least one housing part of the treatment body (2) consists of an opaque material and/or has an opaque surface and/or that at least one light opening is provided in the housing or housing part for light emission radially inwards and/or towards the centre of the treatment body (2).

14. Penis ring according to one of the preceding claims, wherein at least one housing or housing part has, at least in sections, a sheathing (13) and/or coating made of a particularly elastic plastic material, in particular a silicone, rubber, latex and/or polyurethane sheathing and/or coating, preferably wherein the sheathing (13) and/or coating is provided on the treatment body (2) without interruption and/or closed in on itself, with the light opening (11) or light openings (11) being omitted.

15. Penis ring according to claim 14, wherein the sheathing (13) and/or coating is aligned with a light-emitting surface of the light source (10) and/or a surface of the closure element (12) and/or merges smoothly and/or without interruption into these, and/or the surface, preferably the inner ring surface (3), of the treatment body (2) is mechanically and/or chemically modified and/or has the sheathing (13) and/or coating in order to improve the adhesion of at least one, in particular water-based, photosensitiser.

## Revendications

1. Anneau pénien (1), en particulier pour la stimulation sexuelle et/ou pour favoriser l'érection chez l'homme, de préférence pour augmenter l'activité des spermatozoïdes, comprenant un corps de traitement annulaire (2) destiné à entourer le pénis, un dispositif d'irradiation intégré dans le corps de traitement (2) et comprenant au moins un moyen d'éclairage (10), en particulier des sources lumineuses LED, avec une source d'alimentation intégrée pour alimenter le dispositif d'irradiation et avec un circuit de commande pour commander le dispositif d'irradiation, le dispositif d'irradiation étant configuré pour émettre une lumière bleue et/ou bleu-violet dans la gamme de longueurs d'onde visible avec une longueur d'onde comprise entre 410 nm et 450 nm, le dispositif d'irradiation étant configuré pour éclairer avec une intensité d'irradiation comprise entre 0,1 mW/cm(²⁾ et 2,0 mW/cm² et le circuit de commande étant conçu pour couper automatiquement l'irradiation lorsqu'une énergie d'irradiation inférieure à 5,0 J est atteinte et/ou après une durée d'irradiation inférieure ou égale à 60 minutes.

2. Anneau pénien selon la revendication 1, dans lequel le dispositif d'irradiation est configuré pour éclairer avec une intensité d'irradiation comprise entre 0,5 mW/cm² et 1,5 mW/cm² .

3. Anneau pénien selon la revendication 1 ou 2, dans lequel le circuit de commande est conçu pour couper automatiquement l'irradiation lorsqu'une énergie d'irradiation inférieure à 2,0 J, de préférence comprise entre 0,5 J et 1,5 J, et/ou après une durée d'irradiation inférieure ou égale à 30 minutes, de préférence inférieure ou égale à 20 minutes, est atteinte.

4. Anneau pénien selon l'une des revendications précédentes, dans lequel il est prévu un agencement de la source lumineuse (10) et/ou un angle de rayonnement de la source lumineuse (10) qui permet un rayonnement dirigé radialement vers l'intérieur et/ou vers le centre du corps de traitement (2).

5. Anneau pénien selon l'une des revendications précédentes, le dispositif d'irradiation comportant plusieurs sources lumineuses (10), de préférence les sources lumineuses (10) sont disposés dans le sens circonférentiel, de préférence de manière uniformément répartie, le long d'une surface annulaire intérieure du corps de traitement et/ou les sources lumineuses (10) sont disposées de manière e dans le sens circonférentiel, de préférence de manière uniformément répartie, sur toute la surface annulaire intérieure (3) du corps de traitement (2).

6. Anneau pénien selon l'une des revendications précédentes, le corps de traitement (2) présentant de préférence sur la surface annulaire intérieure (3) au moins une ouverture lumineuse (11) pour recevoir de manière particulièrement précise au moins un moyen d'éclairage (10), l'ouverture lumineuse (11) s'étendant de préférence sur une zone de 25 à 75 %, de préférence sur une zone de 40 à 60 %, par rapport à la hauteur axiale du corps de traitement (2), de préférence de la surface annulaire intérieure (3), et/ou l'ouverture lumineuse (11) et/ou une surface de sortie de lumière du moyen d'éclairage (10) s'étendant sur une zone de 25 à 90 %, de préférence de 40 à 60 %, sur la circonférence du corps de traitement (2), de préférence de la surface annulaire intérieure (3), et/ou dans lequel la ou les ouvertures lumineuses (11) sont délimitées et/ou entourées par une surface ou une enveloppe (13) intégrale et fermée du corps de traitement (2).

7. Anneau pénien selon la revendication 6, dans lequel la surface de l'ouverture lumineuse (11) et/ou la surface de sortie de lumière représente ou occupe entre 20 et 80 %, de préférence entre 40 et 60 %, de la surface annulaire intérieure (3) du corps de traitement (2) et/ou dans lequel le corps de traitement (2) est de préférence sur la surface annulaire intérieure (3), présente plusieurs ouvertures lumineuses (11), une ouverture lumineuse (11) séparée étant de préférence attribuée à chaque moyen d'éclairage (10), les ouvertures lumineuses (11) étant de préférence réparties sur toute la circonférence du corps de traitement (2), de préférence sur la surface annulaire intérieure (3), de préférence de manière uniforme.

8. Anneau pénien selon la revendication 7, les ouvertures lumineuses (11) s'étendant au total sur une partie de 30 à 80 %, de préférence de 40 à 70 %, en particulier de 50 à 60 %, le long de la circonférence du corps de traitement (2), de préférence de la surface annulaire intérieure (3), et/ou que la surface de toutes les ouvertures lumineuses (11) représente ou occupe entre 20 et 80 %, de préférence entre 40 et 60 %, d'une surface annulaire (3, 4) du corps de traitement (2), de préférence de la surface annulaire intérieure (3) du corps de traitement (2).

9. Anneau pénien selon l'une des revendications 6 à 8, dans lequel l'ouverture lumineuse (11) et/ou une surface de sortie de lumière du moyen d'éclairage (10) est allongée et/ou en forme de bande et/ou dans lequel l'ouverture lumineuse (11) est ronde, en particulier circulaire, ovale ou ponctuelle et/ou que, de préférence, chaque ouverture lumineuse (11) d' est fermée par un élément de fermeture (10) en particulier en forme de disque, constitué d'un matériau translucide, chaque élément de fermeture (12) présentant de préférence (12) présente une surface satinée et/ou mate et/ou dans lequel de préférence chaque élément de fermeture (12) est constitué d'un matériau transparent et/ou translucide, en particulier en plastique, plus particulièrement en ABS, et/ou présente un tel matériau et/ou dans lequel l'élément de fermeture (12) est conçu comme une lentille convergente ou divergente.

10. Anneau pénien selon l'une des revendications précédentes, dans lequel une surface de sortie de lumière du moyen d'éclairage (10), en particulier d'un élément de fermeture (12), est alignée avec une surface adjacente, en particulier une enveloppe, du corps de traitement (2) et/ou se fond dans celle-ci de manière lisse ou sans interruption et/ou en ce que la source d'énergie est prévue sous la forme d'un accumulateur d'énergie électrique remplaçable et/ou rechargeable, en particulier une pile bouton, et/ou qu'un dispositif de préférence intégré est prévu pour la recharge inductive sans fil de l'accumulateur.

11. Anneau pénien selon l'une des revendications précédentes, dans lequel le corps de traitement (2) est au moins partiellement élastique et/ou flexible dans sa largeur d'ouverture, de préférence élastiquement flexible, réglable et/ou extensible, et/ou dans lequel le corps de traitement (2) présente une interruption (7) s'étendant de préférence sur toute la hauteur axiale du corps de traitement (2).

12. Anneau pénien selon l'une des revendications précédentes, dans lequel le corps de traitement (2) comporte deux languettes de fixation courbées (15, 16) qui se chevauchent, en particulier dans lequel les languettes de fixation (15, 16) sont reliées entre elles de manière amovible, de préférence par complémentarité de forme et/ou par friction, en particulier magnétiquement, et/ou le corps de traitement (2) peut être réglé et/ou élargi de manière élastique dans sa largeur d'ouverture, de préférence en saisissant les extrémités libres ou les pattes de fixation (15, 16).

13. Anneau pénien selon l'une des revendications 11 ou 12, le dispositif de commande comportant un interrupteur de préférence intégré pour activer et/ou désactiver le dispositif d'irradiation, l'interrupteur (14) étant de préférence disposé sur une surface annulaire extérieure (4) du corps de traitement (2), en particulier l'interrupteur (14) étant disposé à l'opposé de l'interruption et/ou des pattes de fixation (15, 16) et/ou en ce qu'un boîtier ou au moins une partie du boîtier du corps de traitement (2) est constitué d'un matériau opaque et/ou présente une surface opaque et/ou en ce qu'au moins une ouverture lumineuse est prévue dans le boîtier ou la partie du boîtier pour une émission de lumière radialement vers l'intérieur et/ou vers le centre du corps de traitement (2).

14. Anneau pénien selon l'une des revendications précédentes, dans lequel au moins un boîtier ou une partie de boîtier présente, au moins par sections, un revêtement (13) et/ou un enrobage en une matière plastique particulièrement élastique, en particulier un revêtement ou un enrobage en silicone, caoutchouc, de latex et/ou de polyuréthane, de préférence l'enveloppe (13) et/ou le revêtement étant prévu(e) sans interruption et/ou fermé(e) sur le corps de traitement (2), à l'exception de l'ouverture de lumière (11) ou des ouvertures de lumière (11).

15. Anneau pénien selon la revendication 14, dans lequel l'enveloppe (13) et/ou le revêtement est aligné avec une surface de sortie de lumière du moyen d'éclairage (10) et/ou une surface de l'élément de fermeture (12) et/ou se fond de manière lisse ou sans interruption dans celle-ci et/ou dans lequel la surface, de préférence la surface intérieure de l'anneau (3), du corps de traitement (2) est modifiée mécaniquement et/ou chimiquement et/ou présente l'enveloppe (13) et/ou le revêtement afin d'améliorer l'adhérence d'au moins un photosensibilisateur, en particulier à base d'eau.
